# EUROPEAN PATENT APPLICATION

(11) **EP 2 706 388 A1**
(43) Date of publication of application: **12.03.2014**
(21) Application number: 13748614.8
(22) Date of filing: 07.02.2013
(51) Int. Cl.: G02B 6/036, A61B 1/00, G02B 6/44, G02B 23/26

(54) **OPTICAL PROBE**

(30) Priority: 14.02.2012 JP 2012029303
(71) Applicant: Sumitomo Electric Industries, Ltd., Chuo-ku Osaka-shi Osaka 541-0041 (JP)
(72) Inventor: HASEGAWA, Takemi, Yokohama-shi, Kanagawa 244-8588 (JP); HIRANO, Mitsuharu, Yokohama-shi, Kanagawa 244-8588 (JP)
(74) Representative: HOFFMANN EITLE
(86) International application number: PCT/JP2013/052816
(87) International publication number: WO 2013/121959

(57) **Abstract**

Provided is an optical probe that can perform low-noise precise OCT measurement even if an object with a high scattering property is measured. The optical probe 20 includes an optical fiber 21 that transmits light between a proximal end 21 a and a distal end 21 b, an optical connector 22 connected with the optical fiber 21 at the proximal end 21 a, a focusing optical system 23 and a deflecting optical system 24 connected with the optical fiber 21 at the distal end 11b, and a support tube 25 and a jacket tube 26 surrounding the optical fiber 21 and extending along the optical fiber 21. The optical fiber 21 includes a core region 41 having a refractive index n₁, a first cladding region 42 surrounding the core region and having a refractive index n₂, a trench region 43 surrounding the first cladding region and having a refractive index n₃, and a second cladding region 44 surrounding the trench region and having a refractive index n₄. The refractive indices have a relationship of n₁ > n₂ > n₃ < n₄.

## Description

### Technical Field

The present invention relates to an optical probe that is used for measuring a tomographic structure of the lumen of an object with a luminal shape such as a blood vessel by using a method of optical coherence tomography (OCT).

### Background Art

U.S. Patent No. 6,445,939 (Patent Literature 1) describes OCT as a method of measuring a tomographic structure of the lumen of an object with a luminal shape, and an optical probe that is inserted into the lumen of the object and used for the OCT measurement. In the OCT measurement, a graded-index optical fiber connected with a tip end (a distal end) of a single-mode optical fiber functions as a lens, and is configured to have a working distance larger than 1 mm and a spot size smaller than 100 µm. Accordingly, an object having an inner radius larger than 1 mm can be optically measured with a spatial resolution smaller than 100 µm.

### Summary of Invention

### Technical Problem

An object of the present invention is to provide an optical probe that can perform low-noise precise OCT measurement even if an object with a high scattering property is measured.

### Solution to Problem

An optical probe of the invention includes an optical fiber that transmits light between a proximal end and a distal end; an optical connector connected with the optical fiber at the proximal end; a focusing optical system having one end connected with the optical fiber at the distal end, the focusing optical system focusing light emitted from the distal end of the optical fiber; a deflecting optical system optically connected with the optical fiber at the distal end, the deflecting optical system deflecting the light emitted from the distal end of the optical fiber; and a jacket tube surrounding the optical fiber, extending along the optical fiber, and being rotatable relative to the optical fiber, the optical connector, the focusing optical system, and the deflecting optical system. The optical fiber includes a core region containing the central axis and having a refractive index n₁, a first cladding region surrounding the core region and having a refractive index n₂, a trench region surrounding the first cladding region and having a refractive index n₃, and a second cladding region surrounding the trench region and having a refractive index n₄. The refractive indices have a relationship of n₁ > n₂ > n₃ < n₄. One end of the deflecting optical system may be connected with the other end of the focusing optical system. Alternatively, a GI lens with one end thereof being obliquely cut may form both the focusing optical system and deflecting optical system.

The optical probe may further include a support tube surrounding the optical fiber, extending along the optical fiber, arranged inside the jacket tube, and fixed to at least a portion of the optical fiber and the optical connector. The support tube may have a structure in which a plurality of linear members are twisted. The support tube may preferably have a thickness of 0.15 mm or larger and a Young's modulus in a range from 100 GPa to 300 GPa. The optical fiber may be preferably configured such that an attenuation of a fundamental mode caused by a bend with a radius of 5 mm is lower than 1 dB/turn and an attenuation of the LP11 mode caused by a bend with a radius of 140 mm is higher than 10 dB/m, with a predetermined wavelength in a range from 1.26 µm to 1.625 5 µm.

### Advantageous Effects of Invention

With the invention, the low-noise precise OCT measurement can be performed even if the object with the high scattering property is measured.

### Brief Description of Drawings

Figure 1 is a conceptual diagram showing an OCT instrument including an optical probe according to a comparative example.
Figure 2 is a conceptual diagram showing an OCT instrument including an optical probe according to an embodiment.

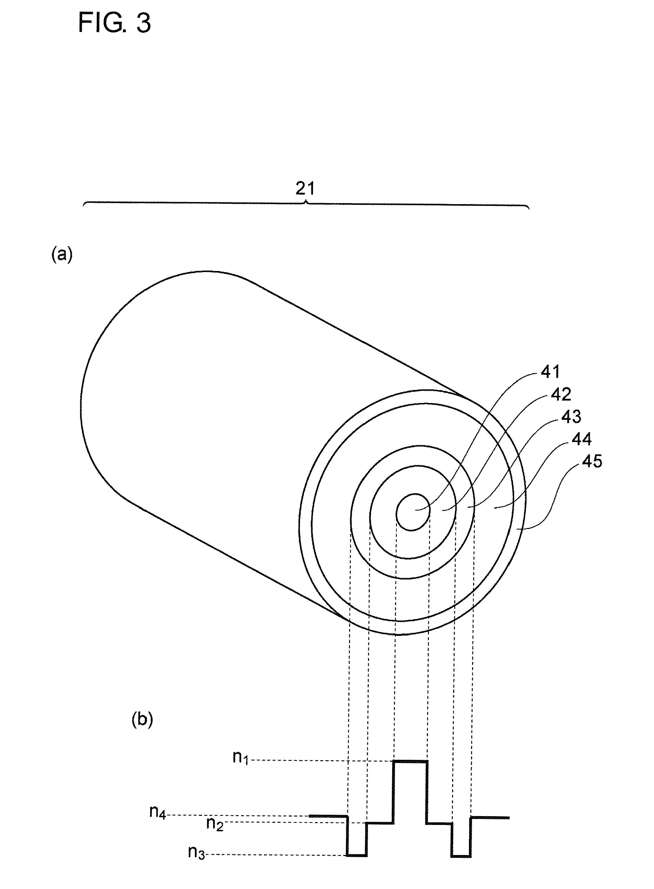

Area (a) of Fig. 3 is a conceptual diagram showing a sectional structure of an optical fiber used for the optical probe according to the embodiment. Area (b) of Fig. 3 is a conceptual diagram showing a refractive index profile of the optical fiber.

### Description of Embodiment

An embodiment for implementing the invention is described below in detail with reference to the attached drawings. It is to be noted that the identical reference sign is applied to the same element in the description for the drawings, and redundant description is omitted. Also, a comparative example is described first, and then an embodiment is described in comparison with the comparative example.

In an OCT instrument using a conventional optical probe, if an object with a high scattering property is to be measured, the inventors have found that noise may be generated by a higher mode of an optical fiber forming the optical probe and precise measurement may not be measured.

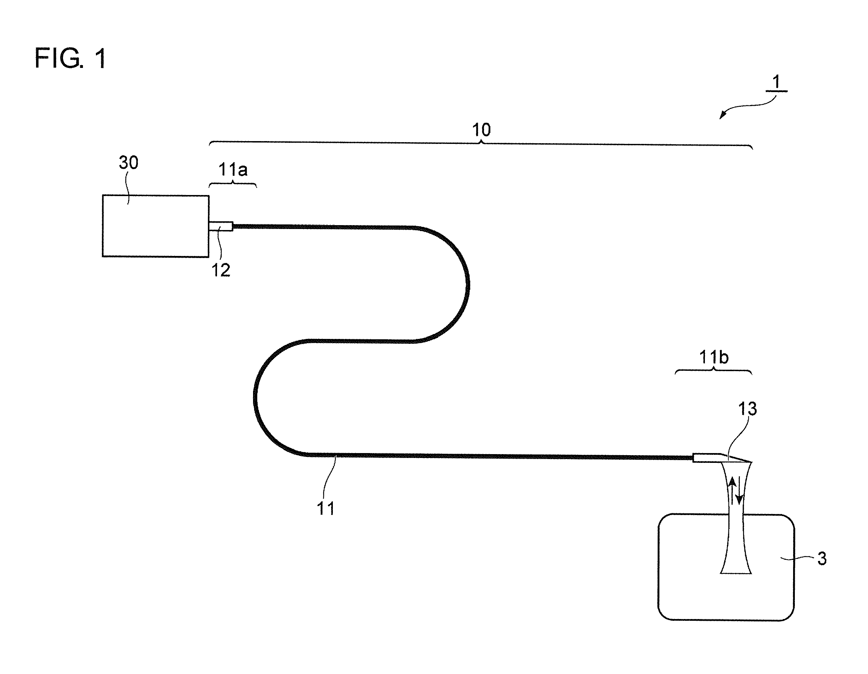

Figure 1 is a conceptual diagram showing an OCT instrument 1 including an optical probe 10 according to a comparative example. The OCT instrument 1 includes the optical probe 10 and a measuring part 30, and acquires an optical coherence tomographic image of an object 3. The optical probe 10 includes an optical fiber 11 that transmits light between a proximal end 11a and a distal end 11b, an optical connector 12 connected with the optical fiber 11 at the proximal end 11a, and an optical system 13 connected with the optical fiber 11 at the distal end 11b. The optical connector 12 is optically connected with the measuring part 30.

Illumination light output from the measuring part 30 is incident on the proximal end 11a of the optical fiber 11 through the optical connector 12, is guided by the optical fiber 11, is emitted from the distal end 11b, and provides irradiation on the object 3 through the optical system 13. Backward reflection light generated in accordance with the irradiation of the illumination light on the object 3 is incident on the distal end 11b of the optical fiber 11through the optical system 13, is guided by the optical fiber 11, is emitted from the proximal end 11a, and is detected by the measuring part 30 through the optical connector 12. The measuring part 30, by analyzing the phase of the backward reflection light, calculates a distribution of backward reflection efficiencies at respective points in the object 3 and acquires a tomographic image of the object 3 based on the calculated distribution.

A mechanism, in which the illumination light emitted from the distal end 11b of the optical fiber 11 reaches the object 3 and returns to the distal end 11b of the optical fiber 11 again, may include reflection, refraction, and scattering. However, the difference among reflection, refraction, and scattering is not essential for the invention, and hence reflection, refraction, and scattering are collectively called backward reflection in the specification for simplifying the description.

In the OCT measurement, the illumination light is emitted as a fundamental mode at the distal end 11b of the optical fiber 11. If the object 3 has a light scattering property, backward reflection light which propagates in various directions is generated at the object 3. In the backward reflection light in the various directions, a component of the backward reflection light which propagates in a direction at about 180 degrees to a propagation direction of the illumination light (a quantitative range depends on a focusing optical system at the tip end of the probe) is coupled with the fundamental mode of the optical fiber 11. The measuring part 30 detects the component of backward scattered light as an OCT signal.

In contrast, in the backward scattered light generated at the object 3, light at an angle, which is formed with respect to the propagation direction of the illumination light, and which is significantly different from 180 degrees, is coupled with the higher mode of the optical fiber 11. If the optical fiber 11 is an ideal single-mode optical fiber, the higher mode is not transmitted by the optical fiber 11, and noise caused by the higher mode is not generated. However, in case of an actual optical fiber, a single-mode property is represented by an attenuation of the higher mode. If the attenuation of the higher mode is not sufficiently high, the magnitude of the higher mode with respect to the magnitude of the fundamental mode becomes not negligible, and the higher mode causes the noise.

The definition of being a single mode and the definition of a cable cut-off wavelength are determined in ITU-T G.650.1 (2004). Referring to this, in a state in which a center part having a length of 20 m of an optical fiber having a length of 22 m is wound with a radius of 140 mm, and each of both ends having a length of 1 m is wound with a radius of 40 mm by one turn, if the attenuation of the LP 11 mode is higher than the attenuation of the LP01 fundamental mode by 19.3 dB, the optical fiber is defined as a single mode. Also, the smallest optical wavelength which provides a single mode is defined as a cable cut-off wavelength. ITU-T G.652 determines an optical fiber with a cable cut-off wavelength of 1.26 µm or smaller as a standard single-mode optical fiber. The definition is also widely used in the market.

However, in actual use during the OCT measurement, the fiber length is markedly smaller than the length of 22 m. During the OCT measurement, the optical path length is measured typically with an accuracy of about 10 µm. Since the temperature dependence of the refractive index of an optical fiber is typically about 10⁻⁵/°C (G. B. Hocker, Applied Optics, Vol. 18, No. 9, pp. 1445-1448 (1979)), even when an optical fiber having a length of 1 m receives a change in temperature by 1°C, an offset of the optical-path length is generated by about 10 µm, and this may non-negligibly affect the measurement of the optical-path length. Hence, the optical-fiber length has to be several meters or smaller. In practical use, for the OCT measurement of a living tissue, the length may be preferably in a range from 2 m to 3 m, the length which is a minimum length required for delivering light between the measuring part 30 and the object 3.

As described above, in actual use of the optical probe for OCT, the fiber lengths is as small as about one-tenth of a length expected by a standard definition for the cut-off wavelength. Owing to this, the attenuation received by the higher mode in the optical fiber is likely decreased. Consequently, even if the optical fiber is the single mode according to the definition, the optical fiber may not be frequently operated with the single mode in actual use during the OCT measurement.

To avoid such a problem, a single-mode optical fiber, which is operated with a single mode in actual use of the optical probe for OCT, has to be selected and used as the optical fiber 11. However, if the higher mode that is dominant in the single mode property has a large bend loss, the fundamental mode also has a large bend loss. Hence, in the single-mode optical fiber used in the conventional optical probe, if the single mode property is provided in actual use of the optical probe for OCT, the bend loss property of the fundamental mode may be increased.

The measuring part 30 may include therein a single-mode optical fiber with a length of about several meters, and occasionally include a higher-mode filter. These elements have an effect of blocking the higher mode. However, the optical probe is rotated at a rotary joint, and the rotary joint actually has a non-zero axial sift and a non-zero angular shift. Hence, mode coupling between the higher mode of the optical probe and the fundamental mode of the optical fiber at the measuring instrument may occur at the rotary joint. In this case, even if the higher mode is blocked in the measuring part 30, the noise caused by the higher mode of the optical probe cannot be restricted.

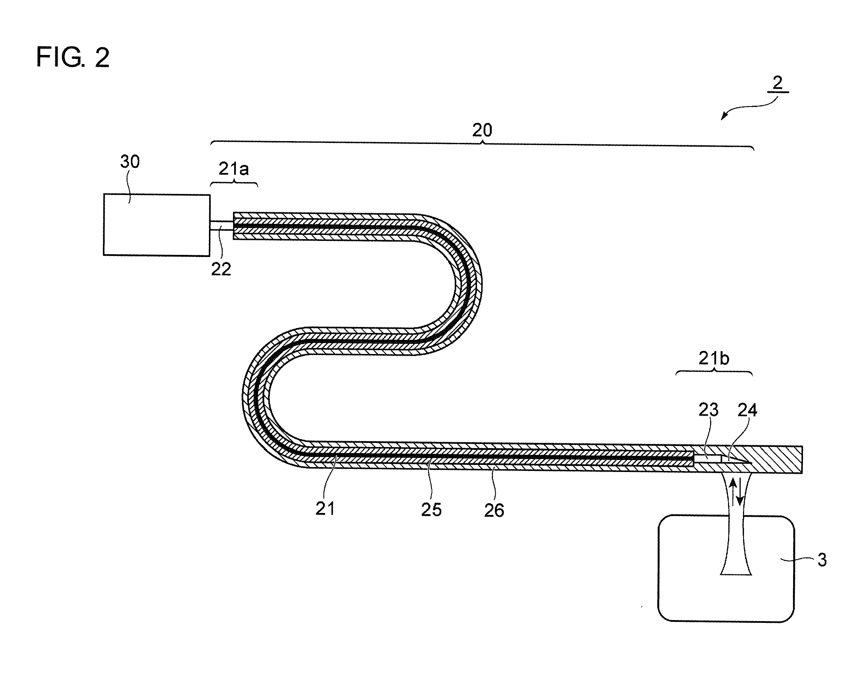

Figure 2 is a conceptual diagram showing an OCT instrument 2 including an optical probe 20 according to an embodiment. The optical probe 20 includes an optical fiber that can restrict generation of higher-mode noise during OCT measurement. The optical probe 20 can also decrease such a potential risk which may be generated by an optical fiber. The OCT instrument 2 includes the optical probe 20 and a measuring part 30, and acquires an optical coherence tomographic image of an object 3. The optical probe 20 includes an optical fiber 21 that transmits light between a proximal end 21a and a distal end 21b, an optical connector 22 connected with the optical fiber 21 at the proximal end 21a, a focusing optical system 23 and a deflecting optical system 24 connected with the optical fiber 21 at the distal end 11b, and a support tube 25 and a jacket tube 26 surrounding the optical fiber 21 and extending along the optical fiber 21. The optical connector 22 is optically connected with the measuring part 30.

A graded index (GRIN) lens serving as the focusing optical system 23, and a mirror serving as the deflecting optical system 24 are connected in series by fusion splicing at the distal end 21b of the optical fiber 21. The focusing optical system 23 focuses the light emitted from the distal end 21b of the optical fiber 21. The deflecting optical system 24 deflects the light emitted from the focusing optical system 23 in the radial direction. Alternatively, a GI lens with one end thereof being obliquely cut may form both the focusing optical system 23 and deflecting optical system 24.

The optical fiber 21 is housed in the inner cavity of the support tube 25. The support tube 25 is fixed to at least a portion of the optical fiber 21 and the optical connector 22. Accordingly, when the optical connector 22 is rotated, the optical fiber 21, the focusing optical system 23, the deflecting optical system 24, and the support tube 25 are rotated together. The optical fiber 21, the focusing optical system 23, the deflecting optical system 24, and the support tube 25 are housed in the inner cavity of the jacket tube 26, and can be rotated in the jacket tube 26. Accordingly, the object 3 can be prevented from being broken because the rotational portion contacts the object 3.

Area (a) of Fig. 3 is a conceptual diagram showing a sectional structure of the optical fiber 21 used in the optical probe 20. Area (b) of Fig. 3 is a conceptual diagram showing a refractive-index distribution of the optical fiber 21. The optical fiber 21 includes a core region 41 containing the central axis and having a refractive index n₁, a first cladding region 42 surrounding the core region 41 and having a refractive index n₂, a trench region 43 surrounding the first cladding region 42 and having a refractive index n₃, a second cladding region 44 surrounding the trench region 43 and having a refractive index n₄, and a coating layer 45 surrounding the second cladding region 44. In the optical fiber 21, the refractive indices of the respective regions have a relationship of n₁ > n₂ > n₃ < n₄ among.

The core region 41, the first cladding region 42, the trench region 43, and the second cladding region 44 have sizes and refractive indices which are adjusted to comply with properties of ITU-T G.657 A or B. Also, the sizes and refractive indices of the regions are each selected so that the attenuation of the LP11 mode with the wavelength of 1.26 µm is 10 dB/m or higher when a bend radius is 140 mm

In general, the attenuation caused by a bend is monotonously increased with respect to the wavelength. Hence, the optical fiber 21 has a sufficiently high attenuation of a higher mode, which is, for example, the LP11 mode, with the wavelength of 1.26 µm or larger. As described in Patent Literature 1, a wavelength of 1.32 µm is typically used in the OCT measurement. Hence, the optical fiber 21 is effective for decreasing the higher-mode noise during the OCT measurement. Such an optical fiber 21 is disclosed in, for example, U.S. Patent Application Publication No. 2009/0279835 as a bend insensitive fiber (BIF fiber).

Also, for the optical fiber 21, the fundamental mode at the wavelength of 1.625 µm may preferably have a bend loss lower than 1 dB/turn with a radius of 5 mm. In general, since the attenuation caused by a bend is monotonously increased with respect to the wavelength, with this optical fiber 21, the bend loss of the fundamental mode is low with a wavelength smaller than the wavelength of 1.625 µm. Accordingly, the optical probe 20 can reach the object 3 through a luminal portion having a curvature radius of about 5 mm, like a branch part of a blood vessel, and the OCT measurement can be performed. With this effect and the bend loss property of the LP11 mode, there is provided the optical probe 20 having small higher-mode noise between the wavelength of 1.26 µm and the wavelength of 1.625 µm, and being applicable to a lumen having a bend. The wavelength band is suitable for OCT measurement of a living tissue because both a light scattering coefficient by a living tissue and light absorption by water are small.

However, if the optical fiber is bent with a curvature radius smaller than 5 mm, a distortion induced by the bend may increase the risk in which the optical fiber is broken. In the single-mode optical fiber used in the conventional optical probe, if a bend which may cause the rupture risk of the optical fiber is generated, the bend loss is increased. Accordingly, an operator of OCT or the measuring instrument could detect generation of a serious bend, and avoid the bend. However, since the optical fiber 21 has a low bend loss, it is difficult to detect and avoid generation of a serious bend, and the rupture risk of the optical fiber 21 is further increased.

Hence, as shown in Fig. 2, the optical fiber 21 may be preferably housed in the inner cavity of the support tube 25. The support tube may preferably have a thickness of 0.15 mm or larger, and a Young's modulus in a range from 100 GPa to 300 GPa, which is similar to that of stainless steel. The support tube 25 may not be necessarily connected in the circumferential direction. The support tube 25 may have a structure in which about 5 to 20 lines are twisted, to adjust flexibility. Accordingly, a small bend, which may increase the rupture risk of the optical fiber 21, can be prevented from being added to the optical probe 20. Such a support tube 25 is disclosed in U.S. Patent Application Publication No. 2002/0151823.

The support tube 25 is fixed to the optical connector 22, and is fixed to at least a portion of the optical fiber 21. Consequently, when the optical connector 22 is rotated, the support tube 25 is rotated together, and a rotation torque is transmitted to the optical fiber 21. Accordingly, as compared with a case in which only the optical fiber 21 is rotated, the torque applied to the optical fiber 21 is decreased, and the optical fiber 21 can be prevented from being ruptured due to the torque.

The GRIN lens serving as the focusing optical system 23 has a refractive-index distribution in which the refractive index is decreased from the center toward the peripheral edge in a section perpendicular to the axis. Also, the mirror serving as the deflecting optical system 24 has a reflection surface at an angle of about 45 degrees with respect to the optical axis. Consequently, the fundamental mode of the optical fmer 21 is focused so that a beam waist is offset from the distal end 21 b by a predetermined distance in the radial direction. The configurations of the lens and mirror are disclosed in Patent Literature 1.

## Claims

1. An optical probe, comprising:
an optical fiber that transmits light between a proximal end and a distal end, an optical fiber including a core region containing the central axis and having a refractive index n₁, a first cladding region surrounding the core region and having a refractive index n₂, a trench region surrounding the first cladding region and having a refractive index n₃, and a second cladding region surrounding the trench region and having a refractive index n₄, the refractive indices having a relationship of n₁ > n₂ > n₃ < n₄;
an optical connector connected with the optical fiber at the proximal end;
a focusing optical system having one end connected with the optical fiber at the distal end, the focusing optical system focusing light emitted from the distal end of the optical fiber;
a deflecting optical system optically connected with the optical fiber at the distal end, the deflecting optical system deflecting the light emitted from the distal end of the optical fiber; and
a jacket tube surrounding the optical fiber, extending along the optical fiber, and being rotatable relative to the optical fiber, the optical connector, the focusing optical system, and the deflecting optical system.

2. The optical probe according to claim 1, wherein one end of the deflecting optical system is connected with the other end of the focusing optical system.

3. The optical probe according to claim 1, further comprising a support tube surrounding the optical fiber, extending along the optical fiber, arranged inside the jacket tube, and fixed to at least a portion of the optical fiber and the optical connector.

4. The optical probe according to claim 3, wherein the support tube has a structure in which a plurality of linear members are twisted.

5. The optical probe according to claim 3, wherein the support tube has a thickness of 0.15 mm or larger and a Young's modulus in a range from 100 GPa to 300 GPa.

6. The optical probe according to claim 1, wherein the optical fiber is configured such that an attenuation of a fundamental mode caused by a bend with a radius of 5 mm is lower than 1 dB/turn and an attenuation of the LP11 mode caused by a bend with a radius of 140 mm is higher than 10 dB/m, with a predetermined wavelength in a range from 1.26 µm to 1.625 µm.
